(19) Europäisches Patentamt
European Patent Office
Office européen des brevets

(11) **EP 3 145 393 B1**

(12) **EUROPEAN PATENT SPECIFICATION**

(45) Date of publication and mention
of the grant of the patent:
**20.11.2019 Bulletin 2019/47**

(51) Int Cl.:
**A61B 5/02** (2006.01)       **A61M 1/34** (2006.01)
**A61M 1/16** (2006.01)

(21) Application number: **15726487.0**

(22) Date of filing: **20.05.2015**

(86) International application number:
**PCT/US2015/031781**

(87) International publication number:
**WO 2015/179523 (26.11.2015 Gazette 2015/47)**

(54) **ABSOLUTE BLOOD VOLUME ESTIMATION USING HEMODILUTION**

SCHÄTZUNG DES ABSOLUTEN BLUTVOLUMENS MITTELS BLUTVERDÜNNUNG

ESTIMATION DU VOLUME SANGUIN ABSOLU PAR HÉMODILUTION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.05.2014 US 201462000684 P**

(43) Date of publication of application:
**29.03.2017 Bulletin 2017/13**

(73) Proprietor: **Fresenius Medical Care Holdings, Inc.
Waltham, MA 02451-1457 (US)**

(72) Inventors:
• **THIJSSEN, Stephan
New York, NY 10044 (US)**
• **KOTANKO, Peter
New York, NY 10128 (US)**

(74) Representative: **Kirkham, Nicholas Andrew et al
Graham Watt & Co. LLP
St. Botolph's House
7-9 St. Botolph's Road
Sevenoaks, Kent TN13 3AJ (GB)**

(56) References cited:
**EP-A1- 2 469 431     US-B1- 7 608 043**

• **W Mcdermott ET AL: "Continuous renal
replacement therapy learning package", , 23
March 2011 (2011-03-23), XP055202606,
Retrieved from the Internet:
URL:http://www.aci.health.nsw.gov.au/__dat
a/assets/pdf_file/0016/220723/CRRT_LP_2010 .p
df [retrieved on 2015-07-15]**

**Description**

BACKGROUND OF THE INVENTION

Fluid overload and anemia play key roles in the morbidity of dialysis patients, and blood volume is a crucial component of these conditions. Knowledge of a patient's absolute blood volume has important ramifications for dry weight assessment, prevention of intra-dialytic complications and anemia management, to name but a few applications.

Therefore there is a need for absolute blood volume estimation in dialysis patients during current clinical practice, on a routine basis, at no significant additional cost, without side effects or radiation exposure, and without changing or significantly interfering with current operational processes.

US 7 608 043 B1 relates to an optical and ultrasound probe for monitoring blood volume changes.

SUMMARY OF THE INVENTION

According to the invention there is provided a hemodialysis machine according to claim 1. Preferred features are provided in the dependent claims.

In one embodiment, a hemodialysis machine includes means for priming a dialyzer with a known volume of priming fluid, means for initiating hemodialysis or ultrafiltration treatment of a patient under conditions whereby the known volume of priming fluid is initially infused into the patient, after which the patient's blood is dialyzed and/or ultrafiltered or both, means for determining the patient's relative blood volume, and means for determining the patient's diluted hemoglobin concentration at a predetermined time, said predetermined time chosen such that systemic hemodilution is caused by priming fluid infusion into the patient while minimizing hemodilution due to recirculated priming fluid. The hemodialysis machine further includes means for comparing the patient's hemoglobin concentration after hemodilution to the patient's pre-dialysis hemoglobin concentration, means for calculating the patient's pre-dialysis absolute blood volume, and means for determining the patient's absolute blood volume periodically during the hemodialysis treatment from the relative blood volume.

Also described although not forming part of the invention is a method of determining the absolute blood volume of a patient undergoing a hemodialysis treatment. The method includes priming a dialyzer with a known volume of priming fluid, initiating hemodialysis treatment of a patient under conditions whereby the known volume of priming fluid is initially infused into the patient, after which the patient's blood is dialyzed or ultrafiltered, determining the patient's relative blood volume, and determining the patient's diluted hemoglobin concentration at a predetermined time, said predetermined time chosen such that systemic hemodilution is caused by priming fluid infusion into the patient while minimizing hemodilution due to recirculated priming fluid. The method further includes comparing the patient's hemoglobin concentration after hemodilution to the patient's pre-dialysis hemoglobin concentration, calculating the patient's pre-dialysis absolute blood volume, and determining the patient's absolute blood volume periodically during the hemodialysis treatment from the relative blood volume.

The present invention enables absolute blood volume estimation in dialysis patients during current clinical practice, on a routine basis, at no significant additional cost, without side effects or radiation exposure, and without changing or significantly interfering with current operational processes.

BRIEF DESCRIPTION OF THE DRAWINGS

The foregoing will be apparent from the following more particular description of example embodiments of the invention, as illustrated in the accompanying drawings in which like reference characters refer to the same parts throughout the different views. The drawings are not necessarily to scale, emphasis instead being placed upon illustrating embodiments of the present invention.

FIG. 1A is an illustration of Bland-Altman plots comparing start-of-dialysis Crit-Line and pre-dialysis Spectra hemoglobin measurements.

FIG. 1B is an illustration of Bland-Altman statistics with limits of agreement (LAG) given at 95% confidence level.

FIG. 2 is an illustration of timing of laboratory results (Spectra) and Crit-Line measurements according to this invention.

FIG. 3 is an illustration of the dilutional effect of static priming fluid addition (240 mL) on Hgb concentration (assuming perfect mixing, negligible Fåhræus effect, UFR 800 mL/h from start of HD, and Crit-Line measurement 5 min into treatment).

FIG. 4 is an illustration of Bland-Altman plots comparing start-of-dialysis CritLine and pre-dialysis Spectra hemoglobin measurements. Top panel: using uncorrected Crit-Line values (identical to FIG. 1A); Middle panel: Crit-Line Hgb corrected for hemodilution (blood volume estimates obtained with Nadler equations); Bottom panel: Crit-Line Hgb corrected for hemodilution (blood volume estimates obtained with Wennesland and Brown equations for males and females, respectively).

FIG. 5 is an illustration of a potential integration of absolute blood volume and pre-dialysis hemoglobin concentration estimates into routine hemodialysis care. For a description of how absolute blood volume can be utilized, see Detailed Description.

## DETAILED DESCRIPTION OF THE INVENTION

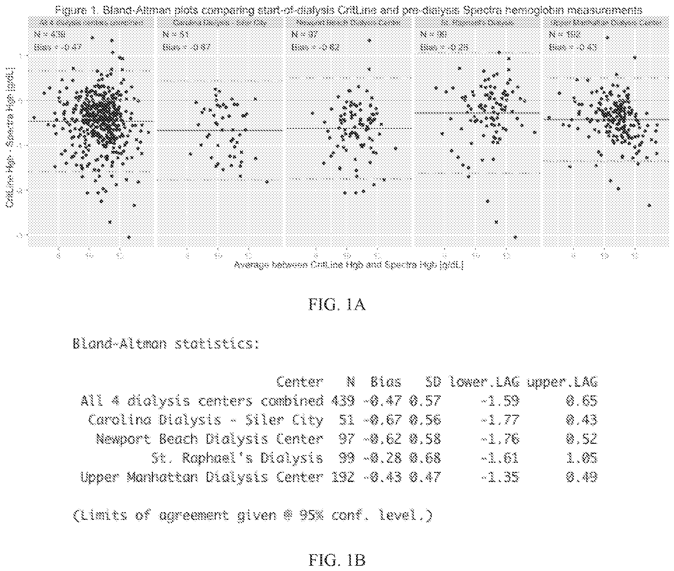

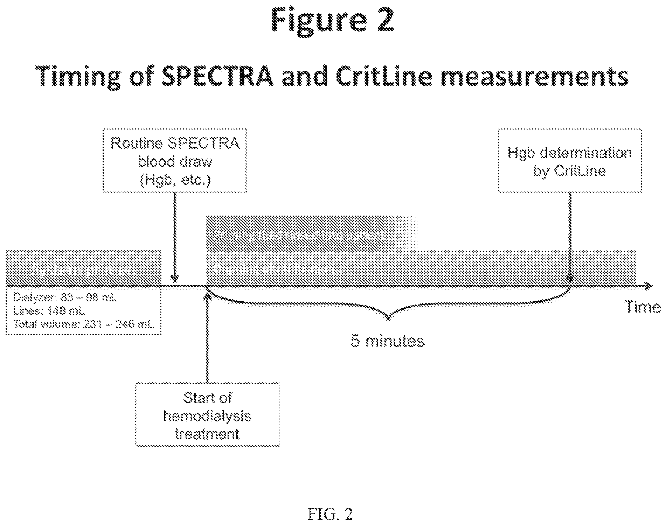

In hemodialysis patients, blood is routinely drawn for laboratory (e.g., Spectra) tests (including hemoglobin (Hgb) concentration) before dialysis and/or ultrafiltration, *i.e.*, the blood is drawn after cannulation of the patient but before the patient is connected to the dialysis machine. Now, with a device such as the Crit-Line monitor (Crit-Line III, Fresenius Medical Care, North America, Waltham MA) that uses photo-optical technology, continuous, non-invasive, real-time measurements of hematocrit (from which Hgb concentration can be derived) can be obtained during hemodialysis and/or ultrafiltration. The first reading is currently obtained approximately 3 to 5 minutes into the dialysis and/or ultrafiltration treatment. This delay is deliberate, so as to ensure that there is no priming fluid recirculation in the extracorporeal circulation at the time of the first Crit-Line Hgb determination. Comparison of this initial Crit-Line Hgb to the pre-dialysis laboratory Hgb revealed that, across different dialysis facilities, the Crit-Line Hgb value is systematically lower than the laboratory result by about 0.5 g/dL on average (see FIGS. 1A and 1B), which is attributed primarily to hemodilution caused by the priming fluid (e.g., saline solution, such as 0.9 wt% NaCl) being flushed into the patient at the start of dialysis. This practice is universal throughout Fresenius hemodialysis clinics in the United States (FIG. 2 illustrates the time course). The volume of priming fluid in the extracorporeal circuit depends on the internal volumes of the blood tubing system and hemodialyzer and is known for any given setup. The priming volume of the blood tubing is approximately 148 mL, while that of the dialyzer depends on the dialyzer model and will vary in the range of between approximately 83 and 98 mL. Thus, the total priming volume will be in the range of between about 231 and 246 mL. This volume is rinsed into the patient over a period of approximately 1.5 minutes at the start of the dialysis and/or ultrafiltration treatment. Therefore, while the delay of the initial Crit-Line Hgb determination avoids any significant hemodilution due to recirculated priming fluid, there will be systemic hemodilution caused by the priming fluid infusion into the patient.

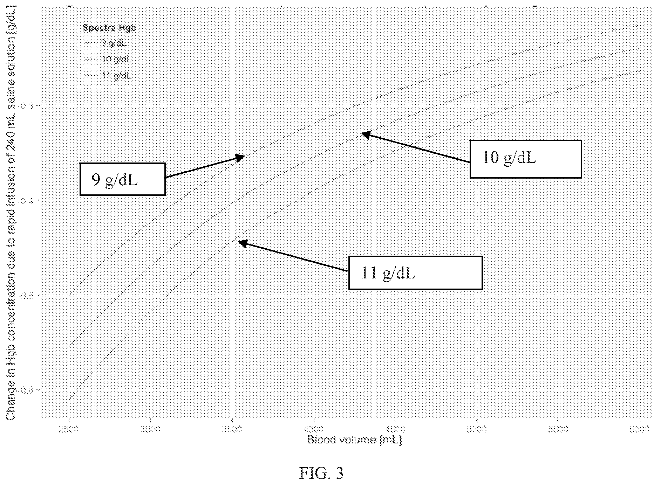

It is assumed that the plasma refill rate and the plasma filtration rate are equal immediately prior to treatment initiation, resulting in no net change of blood volume over time. We further assume that this plasma refill rate does not change between the start of dialysis and the time of hemoglobin measurement by the Crit-line device. Further assuming perfect mixing and no change in fluid distribution between micro- and macrocirculation during the period between start of the treatment and the initial Crit-line Hgb determination the induced change in Hgb concentration due to hemodilution is a function of the absolute blood volume at the start of dialysis, the pre-dialysis Hgb concentration, the amount of priming fluid infused, the half life of the infused priming fluid in the plasma compartment, the time between treatment start and the initial Crit-Line Hgb determination, and the volume ultrafiltered during that time. Pre-dialysis Hgb concentration, delay before the initial Crit-Line measurement, ultrafiltration volume during that time and amount of priming fluid infused are known, the intravascular half life of infused saline solution has been reported in the literature [1], and the change in Hgb concentration can be calculated as the difference between pre-dialysis Hgb and initial Crit-Line Hgb. See FIG. 3 for a graphical representation of these relationships. This permits calculation of the patient's pre-dialysis absolute blood volume as long as $Hgb_{Spectra}$ is not equal to $Hgb_{Critline}$ in accordance with

$$BV = \left(V_{saline} \cdot 0.5^{\left(t_{crit}/t_{\frac{1}{2}}^{saline}\right)} - V_{UF[t_0,\,t_{crit}]}\right) \cdot \frac{Hgb_{CritLine}}{\left(Hgb_{Spectra} - Hgb_{CritLine}\right)} \tag{1.1}$$

with $BV$ being the pre-dialysis blood volume, $V_{saline}$ being the volume of saline in the extracorporeal circuit when connecting the patient to the dialysis machine, $t_{\frac{1}{2}}^{saline}$ being the half life of infused saline solution in the plasma compartment, $V_{UF[t_0,t_{crit}]}$ being the ultrafiltered volume during the interval from the start of dialysis ($t_0$) to the first Crit-Line measurement ($t_{crit}$), $Hgb_{CritLine}$ being the initial Hgb concentration measured by the Crit-Line monitor, and $Hgb_{Spectra}$ being the current pre-dialysis Hgb concentration. The equation yields the pre-dialysis blood volume in the same unit as that used for $V_{saline}$, provided that all Hgb measurements use identical units and $t_{cirt}$ and $t_{\frac{1}{2}}^{saline}$ are expressed in identical units. The half life of infused saline solution in the plasma compartment is about a half an hour [1].

In another embodiment, it may be desirable to choose the minimum $Hgb_{critline}$ as the preferred $Hgb_{critline}$ data point for the computation of BV.

In yet another embodiment it can be desirable to withhold ultrafiltration until $Hgb_{critLine}$ has been measured to achieve a better signal-to-noise ratio by maximizing the degree of hemodilution.

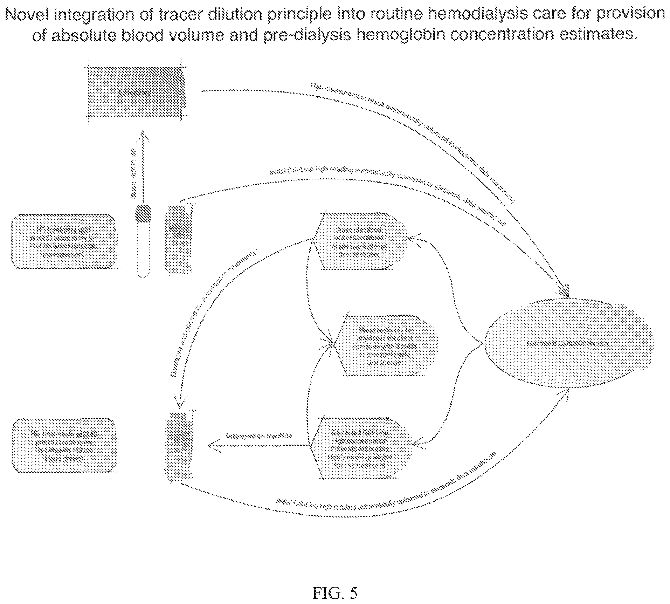

Note that Equation 1.1 reflects a preferred embodiment (using the Crit-Line monitor for intra-dialytic Hgb measurement and a pre-dialysis Hgb concentration reported by Spectra Laboratories), but that neither the equation nor the method depends on the availability of Spectra Laboratories or the Crit-Line monitor, because any precise method of determining Hgb can be used as a substitute. Although not within the scope of the claims, any substance or quantity (*e.g.*, Hematocrit (Hct)) that can reflect hemodilution can be substituted for Hgb. In addition, the priming fluid can be a fluid other than saline, such as, for example, 5% glucose solution or Ringer solution. Further note that the priming fluid is rinsed into the patient over a period of approximately 1 to 1.5 minutes. The net extravasation is, therefore, even a little less than approximated by the above equation. Also, it stands to reason that the plasma half life of infused saline in hemodialysis patients (who are often fluid overloaded) is likely going to be somewhat longer than in healthy controls, further reducing net extravasation. Lastly, note that the Fresenius 2008T dialysis machine will incorporate the Crit-Line monitor, and that in this setting the delay between treatment start and initial Crit-Line measurement will not only be known but will be under the control of the dialysis machine and precisely administered. Both pre-dialysis and Crit-Line Hgb data will be fed into the electronic data warehouse, as shown in FIG. 5. An embodiment as shown in FIG. 5 is, therefore, possible in which the process of blood volume estimation is fully automated, such that whenever a pre-dialysis Hgb value becomes available in the data warehouse, the system extracts the initial Crit-Line Hgb and all other necessary parameters mentioned above for the corresponding treatment and makes a blood volume estimate instantly available in the data warehouse for the physician or technician to review. To reduce variability induced by various sources (patient physiology, measurement precision, operational variability, etc.), a preferred embodiment can utilize a moving average or sequential average of Crit-Line Hgb values (rather than individual values) for blood volume estimation.

Additionally, using the method described above enables the calculation of the estimated offset between start-of-dialysis Crit-Line and pre-dialysis laboratory reference Hgb concentration in accordance with

$$\Delta Hgb = (Hgb_{Spectra} - Hgb_{CritLine}) = \left(V_{saline} \cdot 0.5^{\left(t_{crit}/t_{\frac{1}{2}}^{saline}\right)} - V_{UF[t_0, t_{crit}]}\right) \cdot \frac{Hgb_{CritLine}}{BV} \quad (1.2)$$

which can then be used to correct the measured Crit-Line Hgb concentration for hemodilution according to

$$Hgb_{CritLine}^{corrected} = Hgb_{CritLine} + \Delta Hgb \quad (1.3)$$

Assuming that the blood volume estimate used for this correction is identical to the actual blood volume of the patient at the beginning of this treatment, that both the Crit-Line monitor and the reference laboratory measure Hgb accurately and with perfect precision, that the underlying assumptions of Eq. 1.1 are satisfied, and that the difference between Crit-Line and reference laboratory Hgb concentration is solely the effect of hemodilution, the corrected Hgb concentration obtained in Eq. 1.3 will be identical to the reference laboratory Hgb concentration.

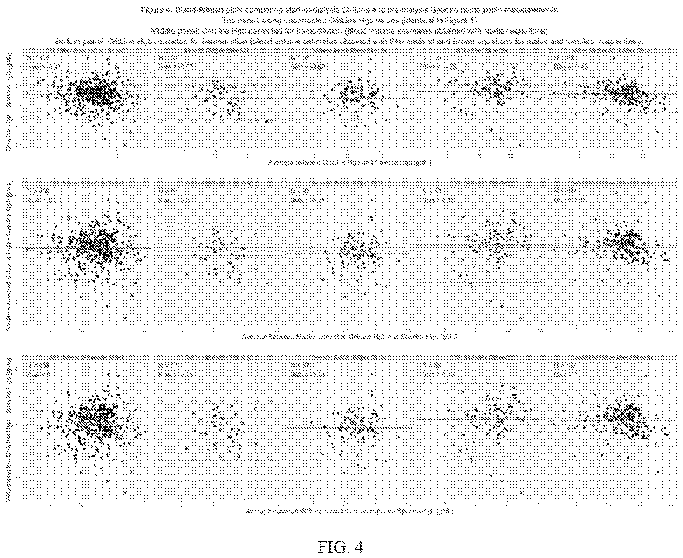

To illustrate the effect of correcting the initial Crit-Line Hgb concentration for hemodilution, the same dataset used for FIG. 1A was extended to include the volume of saline solution administered at the start of the dialysis treatment, the elapsed time of dialysis at the time of the initial Crit-Line Hgb determination, and two separate pre-dialysis blood volume estimates based on published anthropometric equations: 1) the Nadler equations [2] (separate equations for males and females) and 2) the Wennesland equation [3] for males and the Brown [4] equation for females. Based on these additional data, the estimated Crit-Line Hgb offset was calculated for each patient and the Crit-Line Hgb value corrected accordingly. The results are depicted in FIG. 4. For reference purposes, the top panel shows the uncorrected data and is identical to FIG. 1A. The middle panel shows the situation for Crit-Line Hgb values corrected according to BV estimates obtained with the Nadler equations, and the bottom panel utilizes the Wennesland and Brown equations, respectively. Notice how, with appropriate Crit-Line Hgb correction for hemodilution, the bias in the Bland-Altman plots becomes near-zero (Nadler equations) and zero (Wennesland and Brown equations), meaning that with these corrections in place, the corrected Crit-Line Hgb values are on average identical to the reference laboratory values.

In another exemplification, $\Delta Hgb$ was estimated according to Equation 1.2 in 5,731 hemodialysis treatments from 952 Renal Research Institute patients between 3/14/2012 and 9/20/2014, using the following parameters: $Hgb_{Spectra}$ = each patient's pre-dialysis blood Hgb concentration as measured by Spectra Laboratories East, NJ, USA; $Hgb_{CritLine}$ = each patient's blood Hgb concentration as determined by Crit-Line III monitor, averaged between minutes 0 and 2 after initiation of Crit-Line recording; $V_{saline}$ = 250 mL; $t_{crit}$ = 5 min (assuming a typical 4-minute delay between start of hemodialysis and initiation of Crit-Line recording); plasma half-life of infused saline = 20 min [adapted by medical judgment from reference: "Evidence-based Colloid Use in the Critically Ill: American Thoracic Society Consensus Statement", March 2004]; $V_{UF[t0,tcrit]}$ = each patient's cumulative filtration volume between initiation of dialysis and $t_{crit}$, calculated from recorded ultrafiltration rate; BV = each patient's estimated pre-dialysis blood volume, derived by first using the patient's gender, height and post-dialysis body weight to derive an absolute blood volume estimate via the Nadler equation [reference: Nadler SB et al., "Prediction of blood volume in normal human adults"; Surgery, 1962 Feb;51(2):224-32], then back-calculating the start-of-dialysis absolute blood volume based on the change in relative blood volume over the course of the treatment as measured by the Crit-Line III monitor, and then subtracting 250 mL of $V_{saline}$. $Hgb_{CritLine}^{corrected}$ was then calculated according to Equation 1.3. The difference between the two was calculated as

$$Hgb_{offset} = Hgb_{CritLine}^{corrected} - Hgb_{Spectra}.$$ $Hgb_{offset}$ was found to have an average of -0.068 g/dL and a standard deviation of 0.59 g/dL. This result indicates that the corrected Crit-Line Hgb concentration and the measured pre-dialysis Spectra Hgb concentration are nearly identical on average.

A preferred embodiment of this process involves estimation of pre-dialysis blood volume on multiple (ideally closely spaced) occasions on an ongoing basis and using a moving measure for the expected value of blood volume (such as the moving median or moving average) in lieu of a single blood volume determination, so as to reduce the influence of variability in the estimate that is unrelated to physiological differences in blood volume.

A more preferred embodiment can further involve characterizing (*e.g.,* by means of statistical regression) a patient-specific relationship between estimated blood volume and predictor variables (*e.g.,* body weight, serum albumin concentration) and utilizing this relationship to apply a correction to the baseline expected blood volume for each treatment for which a Crit-Line Hgb correction is desired.

Note that, in a properly interconnected IT infrastructure, the process can be entirely automated, resulting in corrected Crit-Line Hgb values being available not only instantly in the data warehouse but also in real-time at the point of care during each dialysis treatment, accessible e.g. via the Crit-Line display.

Note further that, if desired, the impact of the initial saline infusion can be modeled over time to allow the Crit-Line monitor to provide properly corrected Hgb values on an ongoing basis until the hemodilutional effect wears off.

Applications of absolute blood volume (ABV)

1) Display of initial ABV: e.g., HD machine screen, mobile device, clinic client computer
2) Display of current (instantaneous) ABV and relative BV (RBV): e.g. HD machine screen, mobile device, clinic client computer
3) Display of time course of ABV & RBV: e.g. HD machine screen, mobile device, clinic client computer
4) (1) to (3) in relationship to ranges of ABV: e.g. normo-, hyper- and hypovolemia
5) Applications

  a) Fluid management
  b) Prevention of intradialytic complications
  c) Anemia management
  d) Cardiovascular status assessment

5a) The desired ABV can be defined as ABV being in the range of comparable healthy subjects (comparable with respect to age, gender, race, weight, height, other aspects of body composition). Since normal ABV is crucial to maintain adequate perfusion of vital organs and since ABV mediates most of the deleterious effects of fluid overload on the cardiovascular system, ABV can be used to inform decisions on post-HD target and fluid removal.

5b) Intradialytic complications are frequently related to an underfilling of the vascular compartment, i.e. a decrease of ABV below a certain threshold. Knowledge of ABV can be used to alert the staff when this critical threshold is being approached, and/or to automatically implement modifications to treatment and monitoring characteristics (e.g. ultrafiltration rate, treatment time, dialysate temperature, frequency of blood pressure measurements, position of the patient, automatic fluid pulses, change in dialysate conductivity) to avoid intradialytic complications.

5c) ABV is closely related to red blood cell (RBC) mass and thus hemoglobin mass. The goal of anemia management is to bring hemoglobin concentration into a certain target range. However, the hematocrit and concentration of hemoglobin are influenced by dilution. Knowledge of the ABV allows calculating a "normalized" hemoglobin concentration (and hematocrit) and thus allows differentiating between hemodilution and true deficit of hemoglobin. In another application, the ABV is an important component in the mathematical modeling of anemia (refer to PCT Application PCT/US2012/054264 filed on September 7, 2012, published as WO 2013/036836 A2 on March 14, 2013, and U.S. Application No. 14/072,506 published as US2014/0128791 A1 on May 8, 2014).

5d) ABV is a key component of the circulatory system. Knowledge of ABV allows a more comprehensive assessment of cardiovascular function, when used in combination with other indicators, such as heart rate, blood pressures, and cardiac output.

References (numbered in square brackets [#] above)

1. Evidence-based colloid use in the critically ill: American Thoracic Society Consensus Statement. American journal of respiratory and critical care medicine, 2004. 170(11): p. 1247-59.

2. Nadler, S.B., J.H. Hidalgo, and T. Bloch, Prediction of blood volume in normal human adults. Surgery, 1962. 51(2): p. 224-32.

3. Wennesland, R., et al., Red cell, plasma and blood volume in healthy men measured by radiochromium (Cr51) cell tagging and hematocrit: influence of age, somatotype and habits of physical activity on the variance after regression of volumes to height and weight combined. The Journal of clinical investigation, 1959. 38(7): p. 1065-77.

4. Brown, E., et al., Red cell, plasma, and blood volume in the healthy women measured by radiochromium cell-labeling and hematocrit. The Journal of clinical investigation, 1962. 41: p. 2182-90.

An alternative process to estimate pre-dialysis hemoglobin ($Hgb_{Pre}$) level is as follows. Towards the end of the treatment, the relative blood volume as delineated by the Critline monitor ($RBV_{End}$) and the hemoglobin concentration ($Hgb_{End}$) are recorded. The patient's absolute blood volume at the end of the treatment ($ABV_{End}$) can be estimated using empiric formulas, such as the Nadler Equation. The volume of priming fluid infused into the patient at the beginning of the treatment ($V_{saline}$) is recorded. Then, the $Hgb_{Pre}$ is estimated as follows:

$$Hgb_{Pre} = Hgb_{End} * ABV_{End} / (100 * ABV_{End} / RBV_{End} - V_{saline})$$

This equation provides the $Hgb_{Pre}$ in the same unit as that used for $Hgb_{End}$, provided that $ABV_{End}$ and $V_{saline}$ are provided in identical units and $RBV_{End}$ is expressed in %. As an example: $Hgb_{End}$= 10 g/dL; $ABV_{End}$ = 5 L; $RBV_{End}$ = 90%; $V_{saline}$ = 0.25 L $Hgb_{Pre}$ = 10 [g/dL] * 5.0 [L] / (100 * 5.0 [L] / 90 [%] - 0.25 [L]) = 9.42 [g/dL]

This alternative method was exemplified as follows. $Hgb_{pre}$ was estimated according to the equation in paragraph 27 in 5,853 hemodialysis treatments from 952 Renal Research Institute patients between 03/14/2012 and 09/20/2014, using the following parameters: $Hgb_{End}$ = each patient's Hgb concentration as determined by Crit-Line III monitor at the end of dialysis (defined as the median over the last 5 minutes of the dialysis treatment), $ABV_{End}$ = each patient's estimated absolute blood volume at the end of dialysis, estimated based on gender, body height and post-dialysis body weight using the Nadler equation [reference: Nadler SB et al., "Prediction of blood volume in normal human adults"; Surgery, 1962 Feb;51(2):224-32], $RBV_{End}$ = each patient's relative blood volume (expressed in %) as measured by Crit-Line III monitor at the end of dialysis (defined as the median over the last 5 minutes of the dialysis treatment), and $V_{saline}$ = 250 mL.

The difference between $Hgb_{Pre}$ and the measured $Hgb_{Spectra}$ was calculated as $Hgb_{offset} = Hgb_{Pre} - Hgb_{Spectra}$ and was found to have an average of 0.11 g/dL and a standard deviation of 0.6 g/dL.

In one embodiment, end of treatment is defined as the last 5 minutes on dialysis, and $Hgb_{End}$ and $RBV_{End}$ are derived as the median values during this interval. Hgb and RBV do not expect to change materially in that interval.

**Claims**

**1.** A hemodialysis machine comprising:

a) means for priming a dialyzer with a known volume of priming fluid;
b) means for initiating hemodialysis and/or ultrafiltration treatment of a patient under conditions whereby the known volume of priming fluid is initially infused into the patient, after which the patient's blood is dialyzed and/or ultrafiltered;
c) means for determining the patient's diluted hemoglobin concentration at a predetermined time, said predetermined time chosen such that systemic hemodilution is caused by priming fluid infusion into the patient while minimizing hemodilution due to recirculated priming fluid;
d) means for comparing the patient's hemoglobin concentration after hemodilution to the patient's pre-dialysis hemoglobin concentration;
e) means for calculating the patient's pre-dialysis absolute blood volume.

**2.** The hemodialysis machine of claim 1, further comprising:
f) means for determining the patient's relative blood volume.

3. The hemodialysis machine of claim 2, further comprising:
   g) means for determining the patient's absolute blood volume periodically during the hemodialysis treatment from the relative blood volume.

4. The hemodialysis machine of any preceding claim, further comprising means for displaying the pre-dialysis absolute blood volume.

5. The hemodialysis machine of claim 3 further comprising means for displaying the current absolute blood volume and the relative blood volume.

6. The hemodialysis machine of claim 5, further comprising means for displaying a time course of absolute blood volume and relative blood volume.

**Patentansprüche**

1. Hämodialysegerät, das Folgendes umfasst:

   a) Mittel zum Vorbereiten eines Dialysators mit einem bekannten Volumen an Priming-Flüssigkeit;
   b) Mittel zum Einleiten einer Hämodialyse- und/oder Ultrafiltrationsbehandlung eines Patienten unter Bedingungen, bei denen das bekannte Volumen an Priming-Flüssigkeit anfänglich in den Patienten infundiert wird, wonach das Blut des Patienten dialysiert und/oder ultrafiltriert wird;
   c) Mittel zum Bestimmen der verdünnten Hämoglobinkonzentration des Patienten zu einem vorbestimmten Zeitpunkt, wobei der vorbestimmte Zeitpunkt so gewählt ist, dass eine systemische Blutverdünnung durch eine Infusion von Priming-Flüssigkeit in den Patienten verursacht wird, während eine Blutverdünnung aufgrund von rezirkulierter Priming-Flüssigkeit minimiert wird;
   d) Mittel zum Vergleichen der Hämoglobinkonzentration des Patienten nach Blutverdünnung mit der Hämoglobinkonzentration des Patienten vor der Dialyse;
   e) Mittel zum Berechnen des absoluten Blutvolumens des Patienten vor der Dialyse.

2. Hämodialysegerät nach Anspruch 1, das ferner Folgendes umfasst:
   f) Mittel zum Bestimmen des relativen Blutvolumens des Patienten.

3. Hämodialysegerät nach Anspruch 2, das ferner Folgendes umfasst:
   g) Mittel zum periodischen Bestimmen des absoluten Blutvolumens des Patienten während der Hämodialysebehandlung aus dem relativen Blutvolumen.

4. Hämodialysegerät nach einem der vorhergehenden Ansprüche, das ferner Mittel zum Anzeigen des absoluten Blutvolumens umfasst.

5. Hämodialysegerät nach Anspruch 3, das ferner Mittel zum Anzeigen des aktuellen absoluten Blutvolumens und des relativen Blutvolumens umfasst.

6. Hämodialysegerät nach Anspruch 5, das ferner Mittel zum Anzeigen eines zeitlichen Verlaufs des absoluten Blutvolumens und des relativen Blutvolumens umfasst.

**Revendications**

1. Machine d'hémodialyse comprenant :

   a) des moyens d'amorçage d'un dialyseur en utilisant un volume connu de fluide d'amorçage ;
   b) des moyens d'initiation d'une hémodialyse et/ou de traitement par ultrafiltration d'un patient dans des conditions où le volume connu de fluide d'amorçage est dans un premier temps administré par perfusion au patient, après quoi le sang du patient est dialysé et/ou ultrafiltré ;
   c) des moyens permettant de déterminer la concentration en hémoglobine diluée du patient à un moment prédéterminé, ledit moment prédéterminé étant choisi de manière à entraîner une hémodilution systémique par l'administration par perfusion du fluide d'amorçage au patient tout en minimisant l'hémodilution en raison du fluide d'amorçage en recirculation ;

d) des moyens de comparaison de la concentration en hémoglobine du patient après hémodilution à la concentration en hémoglobine du patient avant la dialyse ;

e) des moyens de calcul du volume sanguin absolu du patient avant la dialyse.

2. Machine d'hémodialyse selon la revendication 1, comprenant en outre :
f) des moyens permettant de déterminer le volume sanguin relatif du patient.

3. Machine d'hémodialyse selon la revendication 2, comprenant en outre :
g) des moyens permettant de déterminer le volume sanguin absolu du patient, à partir du volume sanguin relatif, périodiquement pendant le traitement par hémodialyse.

4. Machine d'hémodialyse selon une quelconque revendication précédente, comprenant en outre des moyens d'affichage du volume sanguin absolu avant la dialyse.

5. Machine d'hémodialyse selon la revendication 3, comprenant en outre des moyens d'affichage du volume sanguin absolu réel et du volume sanguin relatif.

6. Machine d'hémodialyse selon la revendication 5, comprenant en outre des moyens d'affichage d'une évolution temporelle du volume sanguin absolu et du volume sanguin relatif.

Figure 1. Bland-Altman plots comparing start-of-dialysis CritLine and pre-dialysis Spectra hemoglobin measurements

FIG. 1A

Bland-Altman statistics:

| Center | N | Bias | SD | lower.LAG | upper.LAG |
|---|---|---|---|---|---|
| All 4 dialysis centers combined | 439 | -0.47 | 0.57 | -1.59 | 0.65 |
| Carolina Dialysis - Siler City | 51 | -0.67 | 0.56 | -1.77 | 0.43 |
| Newport Beach Dialysis Center | 97 | -0.62 | 0.58 | -1.76 | 0.52 |
| St. Raphael's Dialysis | 99 | -0.28 | 0.68 | -1.61 | 1.05 |
| Upper Manhattan Dialysis Center | 192 | -0.43 | 0.47 | -1.35 | 0.49 |

(Limits of agreement given @ 95% conf. level.)

FIG. 1B

9

# Figure 2

## Timing of SPECTRA and CritLine measurements

FIG. 2

FIG. 3

Figure 4. Bland-Altman plots comparing start-of-dialysis CritLine and pre-dialysis Spectra hemoglobin measurements
Top panel: using uncorrected CritLine Hgb values (identical to Figure 1)
Middle panel: CritLine Hgb corrected for hemodilution (blood volume estimates obtained with Nadler equations)
Bottom panel: CritLine Hgb corrected for hemodilution (blood volume estimates obtained with Wennesland and Brown equations for males and females, respectively)

FIG. 4

Novel integration of tracer dilution principle into routine hemodialysis care for provision of absolute blood volume and pre-dialysis hemoglobin concentration estimates.

FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7608043 B1
- US 2012054264 W
- WO 2013036836 A2
- US 072506
- US 20140128791 A1

**Non-patent literature cited in the description**

- *Evidence-based Colloid Use in the Critically Ill: American Thoracic Society Consensus Statement,* March 2004
- **NADLER SB et al.** Prediction of blood volume in normal human adults. *Surgery,* February 1962, vol. 51 (2), 224-32
- Evidence-based colloid use in the critically ill: American Thoracic Society Consensus Statement. *American journal of respiratory and critical care medicine,* 2004, vol. 170 (11), 1247-59
- **NADLER, S.B. ; J.H. HIDALGO ; T. BLOCH.** Prediction of blood volume in normal human adults. *Surgery,* 1962, vol. 51 (2), 224-32
- **WENNESLAND, R. et al.** Red cell, plasma and blood volume in healthy men measured by radiochromium (Cr51) cell tagging and hematocrit: influence of age, somatotype and habits of physical activity on the variance after regression of volumes to height and weight combined. *The Journal of clinical investigation,* 1959, vol. 38 (7), 1065-77
- **BROWN, E. et al.** Red cell, plasma, and blood volume in the healthy women measured by radiochromium cell-labeling and hematocrit. *The Journal of clinical investigation,* 1962, vol. 41, 2182-90
- *Renal Research Institute patients between,* 14 March 2012